# EUROPEAN PATENT APPLICATION

(11) **EP 4 624 587 A1**
(43) Date of publication of application: **01.10.2025**
(21) Application number: 23895077.8
(22) Date of filing: 24.11.2023
(51) Int. Cl.: C12Q 1/06, C12Q 1/24

(54) **RAPID ANTIBIOTIC SUSCEPTIBILITY TEST AND IDENTIFICATION METHOD**

(30) Priority: 24.11.2022 KR 20220158985
(71) Applicant: QuantaMatrix Inc., Seoul 08506 (KR)
(72) Inventor: KIM, Tae Hyun, Gyeongsangnam-do 52849 (KR); KANG, Jun Won, Seoul Seoul 08275 (KR); LEE, Gi yoon, Seoul 08832 (KR); JANG, Hae Wook, Seoul 08788 (KR); JOO, Hyelyn, Namyangju-si Gyeonggi-do 12251 (KR); KIM, Hamin, Seoul 08832 (KR); LIM, Taegeun, Seoul 04421 (KR); KIM, Dongyoung, Seongnam Gyeonggi-do 13597 (KR); KWON, Sunghoon, Seoul 08826 (KR)
(74) Representative: Prüfer & Partner mbB Patentanwälte · Rechtsanwälte
(86) International application number: PCT/KR2023/019090
(87) International publication number: WO 2024/112150

(57) **Abstract**

The present invention relates to a rapid antibiotic susceptibility test and an identification method capable of rapidly providing information on antibiotics suitable for microorganisms by performing an antibiotic susceptibility test and an identification test in a single process. The present invention provides a rapid antibiotic susceptibility test and an identification method comprising the steps of: (a) transferring a sample taken from a patient to a first reaction space in which a sample separation means is present; (b) separating and concentrating the pathogen in the sample from the patient's sample by capturing them with the separation means within the first reaction space; and (c) performing the identification of the pathogen or an antibiotic susceptibility test.

## Description

### [Technical Field]

The present invention relates to a rapid antibiotic susceptibility test and an identification method. More specifically, the present invention relates to a rapid antibiotic susceptibility test and identification method that can provide information on antibiotics suitable for microorganisms in whole blood or other patient-derived samples including urine more quickly by omitting the lengthy blood culture process or minimizing the fungal culture process that was considered essential for antibiotic susceptibility testing and identification processes.

### [Background Art]

In order to prescribe appropriate antibiotics for infections including bacteremia or sepsis, it is necessary to identify the pathogen and test for antibiotic susceptibility to determine whether the causative pathogen is resistant to a certain antibiotic. Many attempts have been made to shorten the time required for antibiotic susceptibility testing because shortening the time required for appropriate antibiotic administration dramatically increases patient survival rates, suppresses the development of antibiotic resistance, and reduces medical costs by shortening the treatment period. The first attempt was to reduce the time required for judgment by improving the method of judging pathogen growth of AST, and several methods such as microscopic imaging method, absorbance measurement method, fluorescent molecular measurement method, electrochemical measurement method, and genomic analysis method produced significant results.

However, although this testing method can reduce the time required for antibiotic susceptibility testing, it still has the limitation of taking a long time to culture the pathogen in the sample extracted from the patient for antibiotic susceptibility testing. Previously, blood cultures from patient's samples were performed for one day, followed by pure cultures for another one day, which took at least two days. To overcome this limitation, there have been attempts to skip the pure culture step required for antibiotic susceptibility testing. Pure culture has the advantage of filtering out human-derived substances with antibiotic activity and a wide range of antibiotics present in the patient's sample when administered to the patient, and of allowing antibiotic susceptibility testing while maintaining the intact health of the pathogen. In addition, it has the function of increasing the reliability of the test by allowing the concentration of the pathogen, which plays an important role in the reproducibility of the antibiotic susceptibility test, to be adjusted to a certain level and then injected into the test. Therefore, if blood culture is used for testing without pure culture, there is a disadvantage in that errors due to the aforementioned factors must be taken into account, but since the need for emergency situations is greatly enhanced by the advantage of shortening the time by more than a day, continuous product development is being carried out. For this product, various techniques are being introduced to compensate for the shortcomings of using blood samples as much as possible, such as testing methods that are less affected by pathogen concentration and deriving results through machine learning.

The present invention proposes a method to minimize the preparation time required for an antibiotic susceptibility test by performing a process for isolating, purifying, concentrating, and efficiently culturing pathogens immediately after collecting a patient's sample (without blood culture or pure culture processes), while blocking the negative effects mentioned above. In other words, the present invention presents a procedure and method including the entire sample-to-answer process from a patient's sample to very rapid, high-sensitivity identification and derivation of antibiotic susceptibility test results.

The reasons why it takes a long time to culture a pathogen from a patient's sample are as follows. 1. The growth of the pathogen is inhibited by blood components and residual antibiotic components administered in the body, and 2. It must go through a division process of about 23 times or more starting from an initial concentration of 10 CFU/ml or less to a final concentration of 10^8 CFU/ml or more. Since the division cycle is different for each pathogen and the time required to reach the required amount is set, it is difficult to shorten the time. According to current routine protocols in diagnostic laboratories, both blood and pure cultures are cultured for a period of time sufficient to allow the pathogen to reach saturation. Therefore, if impurities that interfere with culture are removed early, cultured in a solution optimized for pathogen growth, and cultured samples that are cultured to the minimum concentration required for antibiotic susceptibility are directly used for testing, the testing time can be significantly shortened. To achieve this, a method that can selectively isolate only bacteria is needed, and this selection process is expected to have the effect of concentrating and purifying the bacteria within the sample. Currently, there are many methods available, such as centrifugation methods, filtering methods, and methods of attaching to solid substrates using various probes, but they have limitations in terms of sensitivity and specificity, making it difficult to expect their effectiveness, especially in whole blood samples containing various complex substances.

Accordingly, the present invention proposes a method for rapidly conducting an antibiotic susceptibility test with a small amount of pathogen (before saturation) by selecting and isolating pathogens such as bacteria present in a patient's sample, optimizing the culture environment, and enabling pathogen identification with a small amount of pathogen through sample purification, and further enabling pathogen counting. There are several methods for isolating bacteria, but as an example, the method of utilizing magnetic capture beads coated with special molecular proteins or peptides that can bind to bacteria will be described. In more detail, the steps of collecting a patient's sample, treating the patient's sample with magnetic capture beads to transfer and concentrate a small number of pathogens into a pure/clear culture medium, and measuring the pathogen concentration in real time while culturing in the culture medium can be carried out first, and if necessary, a step of concentrating the pathogen bound to the magnetic capture beads for the washing process can be added. Pathogen identification testing can be performed at the necessary timing after pathogen isolation or after the isolation and culture process. Finally, the present invention proposes a methodology consisting of a step of conducting an antibiotic susceptibility test when the concentrated pathogen reaches a certain number, and a step of reporting the results of the antibiotic susceptibility test, and through this, it aims to show that results can be obtained in a much shorter period of time than before.

### [Disclosure]

### [Technical Problem]

In order to solve the above-mentioned problem, the present invention aims to provide a rapid antibiotic susceptibility test and an identification method that can eliminate the conventional blood culture step, shorten the time required for the step required for pathogen growth including pure culture, and quickly provide information on antibiotics suitable for microorganisms.

### [Technical Solution]

In order to solve the above-mentioned problem, a rapid antibiotic susceptibility test and an identification method are provided, comprising the steps of: (a) transferring a sample taken from a patient to a first reaction space in which a sample separation means is present; (b) separating and concentrating the pathogen in the sample from the patient's sample by capturing the pathogen with the separation means within the first reaction space; and (c) performing the identification of the pathogen or an antibiotic susceptibility test.

In one embodiment, the patient's sample may be whole blood.

In one embodiment, the capture of the pathogen can be accomplished using a substance that can specifically bind to gram-positive bacteria, gram-negative bacteria, and fungi.

In one embodiment, the substance capable of specifically binding may be at least one selected from the group consisting of antibodies, ApoH, aptamers, and polymers.

In one embodiment, the separation means may be a substrate, filter or magnetic particle having a capture molecule.

In one embodiment, after step (b), the method may further comprise a step (b') of culturing the captured pathogen in a culture medium.

In one embodiment, the step (b') may comprise the steps of: monitoring an increase in the concentration of a pathogen in the culture solution; and dividing at least a part or all of the culture solution into a portion of the culture solution for antibiotic susceptibility testing or a portion of the culture solution for pathogen identification testing when the concentration of the pathogen in the culture solution is more than a preset concentration.

In one embodiment, the method may comprise a process of transferring a portion of the culture solution for pathogen identification testing to an identification module when the concentration of the pathogen in the culture solution in the step (b') is 10^3 CFU/ml or more.

In one embodiment, the method may comprise a process of transferring a portion of the culture solution for antibiotic susceptibility testing to a pretreatment module for antibiotic susceptibility testing when the concentration of the pathogen is 10^3 CFU/ml or more.

In one embodiment, it may perform the following steps within the pretreatment module for antibiotic susceptibility testing: i) a step of transferring the portion of the culture solution for antibiotic susceptibility testing to a second reaction space where magnetic particles having capture molecules exist; ii) a step of separating and concentrating the pathogen in the second reaction space; iii) a step of monitoring an increase in the concentration of the pathogen in the portion of the culture solution; iv) a step of transferring the portion of the culture solution to an antibiotic susceptibility test module when the concentration of the pathogen in the portion of the culture solution is 10^3 CFU/ml or more; and v) a step of performing an antibiotic susceptibility test on the pathogen in the portion of the culture solution.

In one embodiment, it may further comprise a step of diluting the portion of the culture solution prior to performing the antibiotic susceptibility test.

In one embodiment, the step (c) may further comprise a step of selecting a test drug to be used in the antibiotic susceptibility test using the results of the pathogen identification test.

In addition, the present invention provides a device for a rapid antibiotic susceptibility test and an identification test comprising: a first reaction space for separating and concentrating a pathogen from a patient's sample; a pathogen identification test module for performing an identification test of the pathogen separated and concentrated in the first reaction space; an antibiotic susceptibility test module for testing the antibiotic susceptibility of the pathogen separated and concentrated in the first reaction space; and a control module for controlling the first reaction space, the pathogen identification test module, and the antibiotic susceptibility test module, and outputting each progress status and result.

In one embodiment, the first reaction space may be manufactured as a cartridge containing 1 to 10 sub-reaction spaces.

In one embodiment, the first reaction space may include one or more sub-reaction spaces loaded with a separation means equipped with capture molecules.

In one embodiment, the antibiotic susceptibility test module may include a pretreatment module for antibiotic susceptibility testing, and the pretreatment module for antibiotic susceptibility testing may include a second reaction space for secondary separation and concentration of the pathogen separated and concentrated in the first reaction space.

In one embodiment, the second reaction space may be manufactured as a cartridge containing 1 to 10 sub-reaction spaces.

In one embodiment, the second reaction space may include at least one sub-reaction space loaded with a separation means equipped with capture molecules.

### [Advantageous Effects]

The rapid antibiotic susceptibility test and identification test method according to the present invention identifies pathogens separated and purified from a sample with high sensitivity by eliminating the blood culture process, and performs antibiotic susceptibility testing with the minimum number of pathogens required for susceptibility testing by promoting the culture process under optimal culture conditions and simultaneously monitoring the culture process. Therefore, it is possible to provide information on antibiotics suitable for microorganisms, enabling rapid and accurate administration of antibiotics.

### [Description of Drawings]

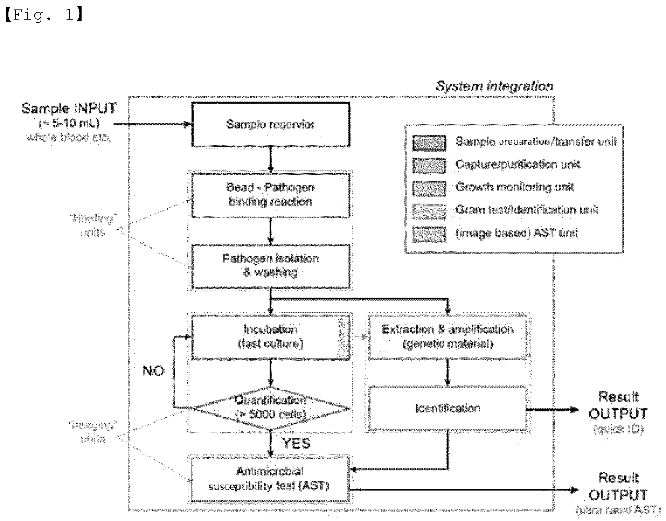

FIG. 1 shows the overall configuration and flow chart of a rapid antibiotic susceptibility test and an identification test method according to one embodiment of the present invention.

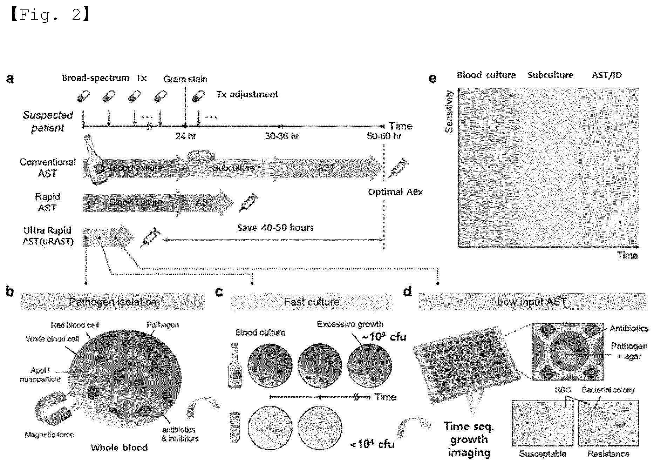

FIG. 2 shows a flow chart of pathogen identification and antibiotic susceptibility testing according to one embodiment of the present invention.

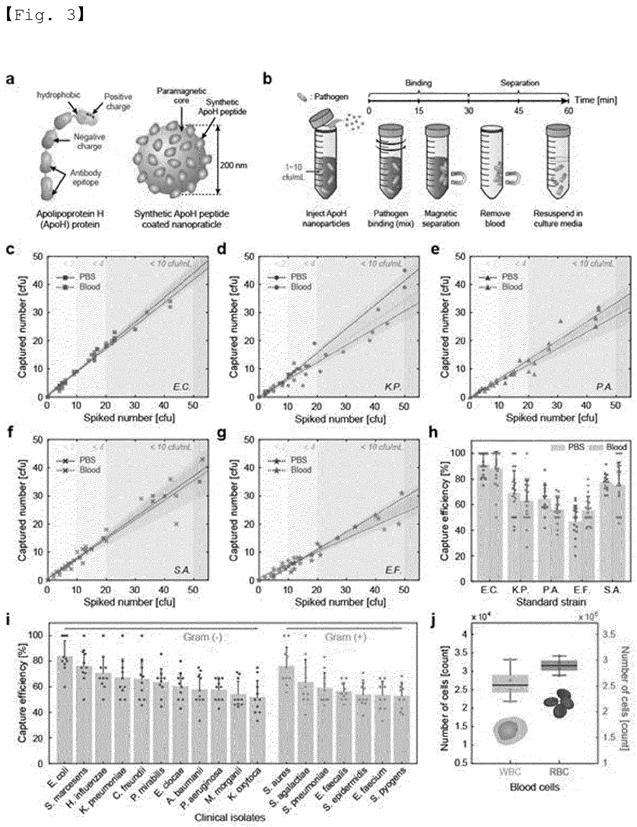

FIG. 3 shows a method and result for separating and purifying a pathogen using magnetic particles according to one embodiment of the present invention.

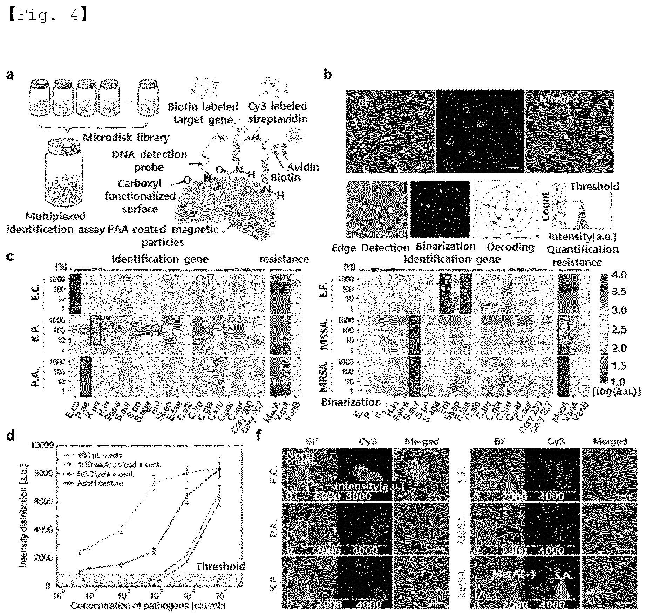

FIG. 4 shows a method for identifying and testing resistance genes for an isolated pathogen according to one embodiment of the present invention, and the results thereof.

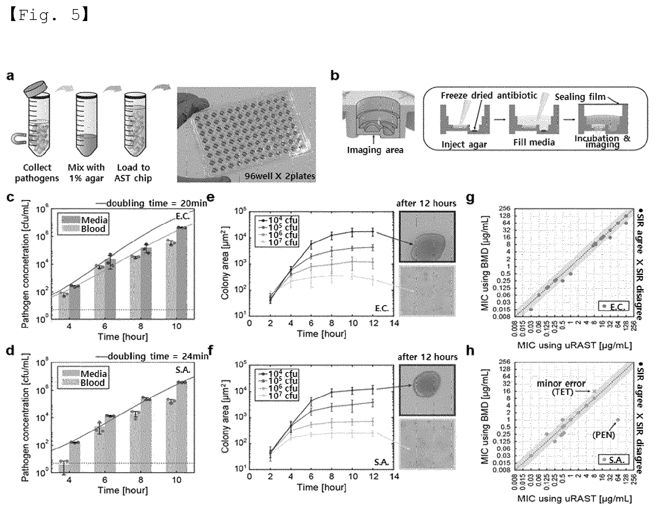

FIG. 5 shows the method and results of an antibiotic susceptibility test according to one embodiment of the present invention.

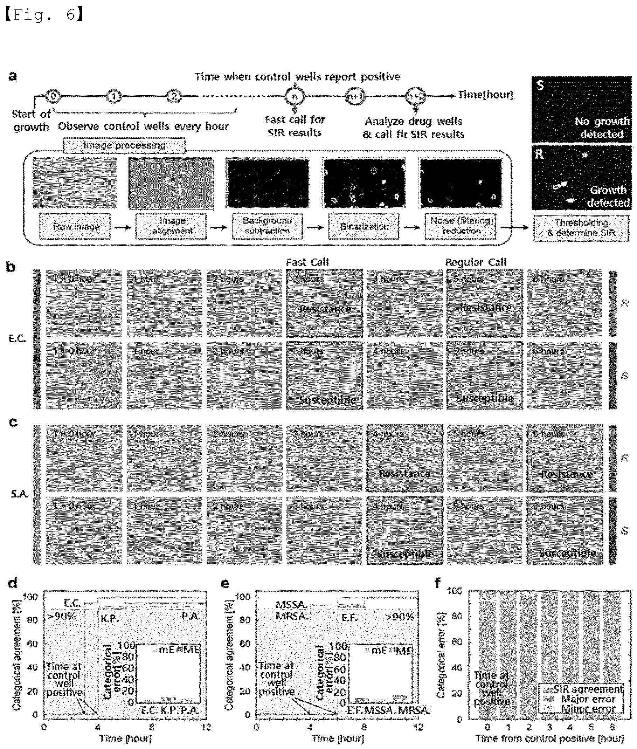

FIG. 6 shows the method and results of an antibiotic susceptibility test according to one embodiment of the present invention.

### [Best Mode]

Hereinafter, preferred embodiments of the present invention will be described in detail. In describing the present invention, if it is judged that a detailed description of related known technologies may obscure the gist of the present invention, the detailed description will be omitted. Throughout the specification, when a part is said to "include" a certain component, this does not mean that other components are excluded, but mean that other components may be included, unless specifically stated otherwise.

The present invention can be modified in various ways and has various embodiments, and specific embodiments are illustrated and described in detail in the detailed description. However, this is not intended to limit the present invention to specific embodiments, but should be understood to include all modifications, equivalents, or substitutes included in the spirit and technical scope of the present invention.

The technology disclosed in this specification is not limited to the implementation examples described herein and may be embodied in other forms. However, the implementation examples introduced herein are provided to ensure that the disclosed content is thorough and complete and to ensure that the technical ideas of the technology can be sufficiently conveyed to those skilled in the art. When describing the drawings as a whole, it is described from the observer's point of view, and when one element is mentioned as being positioned above another element, it includes the meaning that the one element is positioned directly above the other element, or that additional elements may be interposed between the elements. In addition, those skilled in the art will be able to implement the idea of the present invention in various other forms without departing from the technical idea of the present invention.

The terminology used herein is used only to describe specific embodiments and is not intended to limit the present invention. The singular expression should be understood to include the plural expression unless the context clearly indicates otherwise, and the terms "comprises" or "has" and the like are intended to specify the presence of a feature, number, step, operation, component, part or combination thereof described in the specification, and should be understood to not exclude in advance the possibility of the presence or addition of one or more other features, numbers, steps, operations, components, parts or combinations thereof. In addition, in performing the method or manufacturing method, each process constituting the method may occur in a different order from the stated order unless the context clearly states a specific order. That is, each process may occur in the same order as the stated order, may be performed substantially simultaneously, or may be performed in the opposite order.

Meanwhile, the meanings of terms described in this specification should be understood as follows. The term such as "first" or "second" is intended to distinguish one component from another component, and the scope of rights should not be limited by these terms. For example, the first component may be named the second component, and similarly, the second component may also be named the first component.

As used herein, the term 'and/or' includes any combination of multiple listed items or any one of multiple listed items. As used herein, 'A or B' can include 'A', 'B', or 'both A and B'.

The present invention relates to a rapid antibiotic susceptibility test and an identification method comprising the steps of: (a) transferring a sample taken from a patient to a first reaction space in which a sample separation means is present; (b) separating and concentrating the pathogen in the sample from the patient's sample by capturing them with the separation means within the first reaction space; and (c) performing the identification of the pathogen or an antibiotic susceptibility test.

The step (a) is a step for collecting a patient's sample, which is a step for collecting body fluids or secretions to confirm whether the patient has an infection and the type of microorganism. At this time, the body fluid or secretion may include blood, lymph fluid, tears, nasal mucus, saliva, sweat, semen, vaginal discharge, urine, feces, pus, etc., and may not only be simply collected body fluid or secretion, but may also be a part of the body fluid or secretion separated. As an example, the patient's sample may be blood, preferably whole blood, or may be plasma, white blood cells, red blood cells or platelets separated from whole blood.

Additionally, when whole blood is used as the patient's sample, 1 to 10 ml can be used. In the case of a general blood donation, 300 to 500 ml of blood is collected, but in the case of patients undergoing the above test, their physical strength is greatly reduced, so it is necessary to minimize the amount of blood collected as described above. In addition, since whole blood collected from a patient can be used for various tests as well as for identification tests and susceptibility tests like those of the present invention, the less whole blood used in the present invention, the more desirable it is to reduce the burden on the patient. However, as is known, in a typical bacteremia situation, there are about 1 to 10 pathogens present in 1 ml of blood, so a certain amount of whole blood is required to stably secure the pathogen. Therefore, if the whole blood for the above analysis is less than 1 ml, it may be difficult to secure the pathogen stably, and if it exceeds 10 ml, it may be a burden to the patient.

The patient's sample collected as described above can be transferred to the first reaction space in the step (b). The first reaction space is a space where magnetic particles having capture molecules exist, and at this time, the reaction space may simply be a container capable of storing a liquid, such as a vial, beaker, or flask, or a well plate having multiple wells installed. In addition, the first reaction space is in a cartridge format, so that after one test is completed, the reaction space can be removed and a new first reaction space cartridge can be inserted to perform the test. This not only minimizes false positives due to cross-contamination, but also allows for quick preparation for the next test. In addition, in the case of the above cartridge, not only the reaction space but also the magnetic particles and the chemicals necessary to separate the pathogen cells are included, so that a separate preparation process can be omitted. That is, the user of the present invention can prepare the necessary magnetic particles and cell separation agents simply by exchanging the cartridge after one test is completed.

In addition, by further expanding this process, the cartridge may include not only the first reaction space but also a space for isolating the pathogen and a space for culturing the pathogen, which will be described later. In this case, even the cultivation of the pathogen can be performed within one cartridge. In addition, when the culture space of the pathogen is included in the cartridge, it is more preferable that the cartridge includes not only the separation agent but also a medium for culturing the pathogen and a culture agent. Additionally, the above cartridge may include 1 to 10 sub-reaction spaces. In the case of the pathogen separation and concentration, it can be performed in one reaction space, but can also be performed by moving across multiple reaction spaces. Accordingly, in the case of the cartridge, 1 to 10 sub-reaction spaces can be configured, and accordingly, the pathogen separation and concentration process within each reaction space can be smoothly performed. In addition, since the sub-reaction space is located within one cartridge, a new sub-reaction space can be installed and used simultaneously by exchanging the cartridge.

As described above, the sample transferred to the first reaction space can isolate pathogens from a patient's sample.

At this time, a separation means having attached thereto a substance capable of specifically binding to the pathogen may be used, and a substrate, filter or magnetic particle equipped with a capture molecule may be used as a separation means to facilitate the analysis described below.

The capture of the above pathogen can be accomplished using a substance capable of specifically binding to gram-positive bacteria, gram-negative bacteria and fungi, and the substance capable of specifically binding can be at least one selected from the group consisting of antibodies, ApoH, aptamers and polymers. Below, the explanation is centered on using magnetic particles as a separation means and using ApoH as a substance to which it can be specifically bound.

At this time, as examined above, the first reaction space contains magnetic particles inside, and in the case of the present invention, the pathogen can be separated from the sample using these magnetic particles.

Looking at this in more detail, the magnetic particles refer to particles coated with probe materials including proteins, peptides, antibodies, and genes that enable specific binding to a pathogen on the surface of a core made of a magnetic material. At this time, in addition to the above pathogen binding material, a coating process that can prevent defects of non-specific materials can be added. Through this, the pathogen in the blood can be separated. The magnetic particles can be manufactured in various shapes such as spherical or cylindrical. In addition, pathogen separation can also be performed by using various magnetic particles coated with various types of pathogen-binding substances in combination to enable separation of multiple types of pathogens, rather than just one type of magnetic particles. In addition to the pathogen separation method using magnetic particles as an example, a physical method using a filter can also be appropriately used depending on the type of sample. In one embodiment, the magnetic particles perform a pathogen separation process by attaching an Apolipoprotein H (ApoH) protein or an ApoH peptide synthesized with its functional group to a magnetic core.

At this time, the ApoH method is as follows.

### Isolation and purification of apolipoprotein H from human serum albumin

ApoH was purified from human plasma albumin solution as described. Briefly, a human albumin solution from Cohn plasma supernatant IV was applied to a column of cellufine sulfate beads (Chisso co, japan) prebalanced in 0.15 M NaCl. After rinsing with 10 mM phosphate buffer (pH 7.4)-0.2M NaCl, a fraction was eluted with a 2 M NaCl step, then diluted 10-fold in a 10 mM sodium phosphate buffer, (pH 6.8) and loaded over a hydroxylapatite gel (Biorad, USA) prebalanced with the same buffer. The gel was then rinsed with the same buffer and the ApoH eluted by increasing the ionic strength with 1 M KC1. The solution obtained was dialyzed against distilled water and lyophilized. At this stage, the purity of ApoH provided a single band at 50 kDa, as checked by sodium dodecyl sulfate-polyacrylamide gel electrophoresis (SDS-PAGE, 10%). The Apoh thus purified is coupled to nanomagnetic beads (ApoH-Technologies, France).

When a sample is injected into the above reaction space, a pathogen can be attached to the surface of the magnetic particles. Therefore, the sample mixed with magnetic particles in the above reaction space can be stirred in a thermomixer or shaking incubator for 15 to 30 minutes to promote attachment of the pathogen as described above. This stirring can also be performed by supplying a magnetic field from the outside to move the magnetic particles up and down or left and right, and this can also be implemented with a microfluidic chip. In addition, to enable rapid movement of the magnetic particles, the reaction space is reversed up and down 1 to 5 times, allowing mixing of the pathogen and the magnetic particles.

After mixing is completed as described above and the pathogen is attached to the surface of the magnetic particles, the magnetic particles can be separated from the sample. At this time, since the magnetic particles have magnetism, they can be attached to the side or bottom within the reaction space using a magnet. In addition, a magnetic field can be formed at the bottom of the reaction space to fix the magnetic particles at the bottom of the reaction space. The remaining sample can then be discharged to separate the magnetic particles. This separation method can be appropriately selected depending on the structure of the reaction space and cartridge. Preferably, when using multiple reaction spaces (sub-reaction spaces) within the cartridge, the reaction can be performed by moving magnetic particles using the magnet or a device or material that generates magnetism, and when using one sub-reaction space within the cartridge, the magnetic particles can be separated by forming a magnetic field at the side or bottom of the sub-reaction space.

The magnetic particles separated from the sample as described above can be washed 1 to 10 times as needed. Even after the magnetic particles are separated, the sample components such as white blood cells, red blood cells, and platelets that are non-specifically attached to the magnetic particles other than the pathogen may remain on the surface, and if these are cultured and tested as is, the sample components may act as foreign substances, reducing the reliability of the test. Therefore, to minimize these sample components, the magnetic particles can be washed 1 to 10 times as needed. If washing exceeds 10 times, the time and reagents used for testing will increase, and pathogens attached to the surface of the magnetic particles may also be washed away, which may reduce testing accuracy. The above washing can be performed by repeatedly injecting and discharging a washing solution into the reaction space when using one reaction space, and can be performed by sequentially moving the magnetic particles into the reaction spaces containing the washing solution when using multiple reaction spaces.

After the step (b), the method may further comprise a step (b') of culturing the captured pathogen in a culture medium. Although additional culture of the pathogen is performed in subsequent stages depending on the needs of each test, performing each culture may result in significant waste of culture medium and space, which may increase test costs. Therefore, it is possible to reduce the overall test cost and time by isolating the pathogen in step (b) above and then culturing it to a certain concentration. Such additional culture is possible even while attached to the magnetic particles, and additional culture can also be performed separately after the pathogen and the magnetic particles are separated.

In addition, the step (b') may comprise the steps of: monitoring an increase in the concentration of the pathogen in the culture solution; and dividing at least a part or all of the culture solution into a portion of the culture solution for antibiotic susceptibility testing or a portion of the culture solution for pathogen identification testing when the concentration of the pathogen in the culture solution is higher than a preset concentration.

The step of monitoring the increase in the concentration of the pathogen can use an optical method, the same as the monitoring method of step (g) described below.

Through the monitoring described above, if the concentration of the pathogen in the culture solution becomes higher than a preset concentration, at least a portion or all of the culture solution can be divided as a portion of the culture solution for antibiotic susceptibility testing or a portion of the culture solution for pathogen identification testing. That is, the portion of the culture solution is divided into a portion of the culture solution for antibiotic susceptibility testing or a portion of the culture solution for pathogen identification testing and used in each step.

As described above, the pathogen attached to the surface of the magnetic particles can be injected into the culture solution without washing or after washing is completed. Afterwards, through step (c), a portion of the culture solution for identity testing and a portion of the culture solution for antibiotic susceptibility testing can be separated and transferred to each device. For identification and susceptibility tests, steps (e) and (f-h) can be performed independently in the second reaction space and the third reaction space, respectively, simultaneously.

In the pathogen identification test module of the step (e), the following steps can be performed: i) a step of transferring a portion of the culture solution for the identification test to a second reaction space; ii) a step of washing the pathogen in the second reaction space; iii) a step of lysing the pathogen in the portion of the culture solution; iv) a step of tagging and amplifying the extracted gene, and v) a step of performing an identification test for the pathogen through a fluorescence signal after reacting the pathogen gene with an encoded microbead particle coated with a complementary probe gene.

In one embodiment, in the case of the identification test, the culture solution for the identification test separated through step (c) is transferred to the second reaction space, the pathogen is lysed, and the genetic region including the type of pathogen and whether or not it has resistance is tagged and amplified. At this time, the pathogen containing magnetic particles can be washed 1 to 10 times as needed for the purpose of removing factors that interfere with the PCR process in a similar manner to step (b) prior to amplifying the above sample. In addition, in the case of identification testing, testing is possible with pathogen counts of 1 to 5 CFU or less, but in this case, for a more stable test, it is also possible to perform step (e) after additional culture as in the antibiotic susceptibility test described later. In this case, the number of pathogens required for antibiotic susceptibility testing is not used, so it can be completed within a short period of time (1 to 2 hours). The above amplified genes indicating identification and antibiotic resistance can be mixed and bound to various types of bead particles on which each target gene probe is loaded, and by adding fluorescent particles, the presence or absence of a pathogen in the sample, whether it is gram-positive or gram-negative, and the identification and resistance genes of the pathogen can be confirmed. The types of beads that can detect the identification and resistance genes of the pathogen can be identified through codes patterned on the surface of more than 200 types (multiplexity). The results of this pathogen identification test are transferred to the antibiotic susceptibility test module described later, so that testing can be performed using appropriate reagents and antibiotics.

In the pretreatment module for antibiotic susceptibility testing in the steps (e) and (g), the following steps can be performed: i) a step of transferring the portion of the culture solution for antibiotic susceptibility testing to a third reaction space; ii) a step of culturing the pathogen in the third reaction space; iii) a step of monitoring an increase in the concentration and total number of the pathogen in the portion of the culture solution; iv) a step of transferring the portion of the culture solution to an antibiotic susceptibility test module when the concentration of the pathogen in the portion of the culture solution becomes 10^3 CFU/ml or more; and v) a step (h) of performing an antibiotic susceptibility test on the pathogen in the portion of the culture solution.

Unlike the identification test that can be performed with a small number of pathogens, the antibiotic susceptibility test generally requires a large number of pathogens to test for various types and concentrations of antibiotics. Therefore, the pathogen separated from the magnetic particles transferred to the third reaction space specified above can be cultured in an appropriate environment until a certain concentration is reached through step (f) to form a culture solution. At this time, the culture may be performed in a small amount of culture medium suitable for test and culture, and additional culture solution may be supplied as needed, and at this time, the pathogen may be cultured to be a concentration of 10^3 CFU/ml or more. In addition, the culturing process may include a process for isolating a pathogen defective in a magnetic particle to facilitate the growth of the pathogen. At this time, it is preferable to use a liquid medium for the culture medium to facilitate handling, and specifically, the liquid medium may include a natural medium optimized for the type of pathogen, a synthetic medium with added nutrients, a restricted medium, a complex medium, etc. In this process, the effect of concentrating the pathogen can be achieved while simultaneously increasing the number and concentration of the pathogen through the culturing process. The culturing can be performed within a culturing space configured to allow gas exchange, and the culturing space can be included in the cartridge or installed separately from the cartridge, and can include a heating unit to form an appropriate temperature. Additionally, for anaerobic strains, the culture effect can be optimized by supplying separate carbon dioxide.

As described above, the pathogen separated from the third reaction space is in a purified state with the impurities (In the case of whole blood, it includes blood cells) contained in the sample removed, and exists in a transparent culture solution, so it can be monitored optically through step (g). Since the division and growth rates are different depending on the type of isolated pathogen, instead of the conventional blood culture method that requires culturing with an unnecessarily large number of pathogens, performing the test by optically observing when the minimum number of pathogens required for antibiotic susceptibility testing is reached regardless of the pathogen type can have the effect of reducing the test time to the shortest possible time. At this time, the culture solution can be cultured to have a pathogen concentration of 10^3 CFU/ml or more according to the kit to be used for the antibiotic susceptibility test described later. This monitoring step can measure and display the amount of pathogen in the culture solution in real time to confirm whether the pathogen is being cultured appropriately, and can also enable separation and transfer of the culture solution at an appropriate time. At this time, the pathogen concentration can be measured using optics, laser or ultrasound, and any method capable of measuring the pathogen in the culture medium can be used without limitation. In one embodiment, a holographic imaging method was used, but other optical methods can be used alternatively. Specifically, a part or all of the space where the culture solution exists can be made optically transparent, and in particular, a flat structure can be installed in the optically transparent part to facilitate laser transmission. In the case of the culture solution, since it is mixed and heated at regular intervals for the efficiency of culture, a culture solution having the same concentration as the culture solution may exist inside the structure, and by periodically irradiating the structure with a laser and measuring changes in amplitude or phase, the concentration of pathogens in the culture solution can be measured. In addition, during the monitoring, by measuring the pathogen concentration at the beginning of the culture and then measuring the pathogen concentration at each time point and comparing them, it is possible to notify when the desired pathogen concentration is reached.

After culture is completed in the third reaction space as described above, the portion of the culture solution can be transferred to the antibiotic susceptibility test module and an antibiotic susceptibility test can be performed through step (h). At this time, in the case of the antibiotic susceptibility test, it is desirable to use appropriate reagents and antibiotics based on the results of the pathogen identification test. In one embodiment, for antibiotic susceptibility testing, the cultured pathogen is mixed with a heated and liquefied agar solution and dispensed into a cartridge for antibiotic susceptibility testing, and the test results are derived through imaging over time.

At this time, a process of separating and washing the pathogen from the magnetic particles may be added as needed. To this end, the culture solution may be washed with a solution or culture medium having a characteristic composition, and in this process, the bond between the substance on the surface of the magnetic particles and the pathogen may be weakened, allowing the pathogen to be separated from the magnetic particles. In order to recover the pathogen that has fallen off from the magnetic particles, the same method as the pathogen separation process in the first reaction space is used, but here, the supernatant can be recovered and used to recover the pathogen that has fallen off from the magnetic particles. Methods for isolating a pathogen may include applying a physical force, such as vibration, and adding a chemical substance that can cut off the binding site between magnetic particles and the pathogen.

The antibiotic susceptibility test module has a well plate composed of multiple wells, and the cultured culture solution can be injected into the well plate to perform an antibiotic susceptibility test. At this time, 1 to 4 sub-wells can be formed inside the well plate, and the culture solution can be injected into one of these sub-wells. An antibiotic selected based on the results of the pathogen identification test is injected or solidified in another one of the sub-wells or in an independent space of the sub-well into which the culture solution is injected, and after the culture solution is injected, by injecting saline solution or culture medium into the antibiotic, the antibiotic can be dissolved and come into contact with the culture solution. At this time, contact between the culture solution and the antibiotic can be performed through one point or surface, and it is possible to examine the susceptibility of the antibiotic by observing the reaction of the pathogen in the culture medium that has come into contact with the antibiotic at this contact point or surface.

In the case of the present invention, the identification test of the pathogen and the antibiotic susceptibility test are performed by eliminating the blood culture process that was considered essential, and by optimizing and minimizing the isolation of the pathogen in whole blood and the culture process after isolation, the test can be completed in a shorter period of time than with existing methods. In addition, in the case of the present invention, the culture, pathogen identification test, and antibiotic susceptibility test can be performed in an integrated manner. In the case of the antibiotic susceptibility test, since the type of reagent and antibiotic must be determined based on the results of the pathogen identification test after the pathogen identification test is performed, in the existing method, this was performed in a time series manner, but in the case of the present invention, the identification test is performed using some of the isolated pathogens, and the remainder is cultured during the pathogen identification test time, so the test can be completed at a faster rate than in the existing method. In the existing method of collecting a portion of the culture fluid after blood culture and transferring and supplying it to a pathogen identification tester and an antibiotic susceptibility tester to perform each test, there are cases where the results are erroneous due to contamination resulting from dispensing and injection of the culture fluid. However, in the case of the present invention, the culture, pathogen identification test, and antibiotic susceptibility test can be installed in a single device, which means that the results of the pathogen identification test and the antibiotic susceptibility test can be output simply by injecting a patient sample into the device. In addition, in the case of the present invention, a cartridge is used in the culture step, and in the case of the pathogen identification test and the antibiotic susceptibility test, a cartridge-type device such as a well plate is used. Therefore, errors due to cross-contamination can be minimized by replacing all parts that came into contact with the culture solution with a new cartridge after one test is completed.

In addition, in the case of the above antibiotic susceptibility test and pathogen identification test, it is possible to conduct them simultaneously, but it is also possible to perform each part independently. Also, even if they are done simultaneously, they can be done sequentially or simultaneously.

As described above, specific parts of the present invention have been described in detail, so it will be obvious to those skilled in the art that such specific descriptions are merely preferred embodiments and that the scope of the present invention is not limited thereby. Accordingly, the substantial scope of the present invention will be defined by the appended claims and their equivalents.

## Claims

1. A rapid antibiotic susceptibility test and an identification method, comprising the steps of:
(a) transferring a sample taken from a patient to a first reaction space in which a sample separation means is present;
(b) separating and concentrating the pathogen in the sample from the patient's sample by capturing it with the separation means within the first reaction space; and
(c) performing the identification of the pathogen or an antibiotic susceptibility test.

2. The rapid antibiotic susceptibility test and the identification method of claim 1, wherein the patient's sample is whole blood.

3. The rapid antibiotic susceptibility test and the identification method of claim 1, wherein the capture of the pathogen uses a substance that can specifically bind to gram-positive bacteria, gram-negative bacteria, and fungi.

4. The rapid antibiotic susceptibility test and the identification method of claim 3, wherein the substance capable of specifically binding is at least one selected from the group consisting of antibodies, ApoH, aptamers and polymers.

5. The rapid antibiotic susceptibility test and the identification method of claim 1, wherein the separation means is a substrate, filter or magnetic particle having a capture molecule.

6. The rapid antibiotic susceptibility test and the identification method of claim 1, further comprising, after the step (b), a step of (b') culturing the captured pathogen in a culture solution.

7. The rapid antibiotic susceptibility test and the identification method of claim 6, wherein the step (b') comprises the steps of:
monitoring an increase in the concentration of the pathogen in the culture solution; and
dividing at least a part or all of the culture solution into a portion of the culture solution for antibiotic susceptibility testing or a portion of the culture solution for pathogen identification testing when the concentration of the pathogen in the culture solution is higher than a preset concentration.

8. The rapid antibiotic susceptibility test and the identification method of claim 6, which comprises a process of transferring a portion of the culture solution for pathogen identification testing to an identification module when the concentration of the pathogen in the culture solution in the step (b') is 10^3 CFU/ml or more.

9. The rapid antibiotic susceptibility test and the identification method of claim 6, which comprises a process of transferring a portion of the culture solution for antibiotic susceptibility testing to a pretreatment module for antibiotic susceptibility testing when the concentration of the pathogen is 10^3 CFU/ml or more.

10. The rapid antibiotic susceptibility test and the identification method of claim 9, which comprises the following steps performed within the pretreatment module for antibiotic susceptibility test:
i) a step of transferring the portion of the culture solution for antibiotic susceptibility testing to a second reaction space where magnetic particles having capture molecules exist;
ii) a step of separating and concentrating the pathogen in the second reaction space;
iii) a step of monitoring an increase in the concentration of the pathogen in the portion of the culture solution;
iv) a step of transferring the portion of the culture solution to an antibiotic susceptibility test module when the concentration of the pathogen in the portion of the culture solution is 10^3 CFU/ml or more; and
v) a step of performing an antibiotic susceptibility test on the pathogen in the portion of the culture solution.

11. The rapid antibiotic susceptibility test and the identification method of claim 10, further comprising a step of diluting the portion of the culture solution prior to performing the antibiotic susceptibility test.

12. The rapid antibiotic susceptibility test and the identification method of claim 1, wherein the step (c) further comprises a step of selecting a test drug to be used in the antibiotic susceptibility test using the results of the pathogen identification test.

13. A device for a rapid antibiotic susceptibility test and an identification test, comprising:
a first reaction space for separating and concentrating a pathogen from a patient's sample;
a pathogen identification test module for performing an identification test of the pathogen separated and concentrated in the first reaction space;
an antibiotic susceptibility test module for testing the antibiotic susceptibility of the pathogen separated and concentrated in the first reaction space; and
a control module for controlling the first reaction space, the pathogen identification test module, and the antibiotic susceptibility test module, and outputting each progress status and result.

14. The device for a rapid antibiotic susceptibility test and an identification test of claim 13, wherein the first reaction space is manufactured as a cartridge containing 1 to 10 sub-reaction spaces.

15. The device for a rapid antibiotic susceptibility test and an identification test of claim 14, wherein the first reaction space includes one or more sub-reaction spaces loaded with a separation means equipped with capture molecules.

16. The device for a rapid antibiotic susceptibility test and an identification test of claim 13, wherein the antibiotic susceptibility test module includes a pretreatment module for antibiotic susceptibility testing, and
the pretreatment module for antibiotic susceptibility testing includes a second reaction space for secondary separation and concentration of the pathogen separated and concentrated in the first reaction space.

17. The device for a rapid antibiotic susceptibility test and an identification test of claim 16, wherein the second reaction space is manufactured as a cartridge containing 1 to 10 sub-reaction spaces.

18. The device for a rapid antibiotic susceptibility test and an identification test of claim 17, wherein the second reaction space includes at least one sub-reaction space loaded with a separation means equipped with capture molecules.
